# EUROPEAN PATENT APPLICATION

(11) **EP 4 230 158 A1**
(43) Date of publication of application: **23.08.2023**
(21) Application number: 22157893.3
(22) Date of filing: 22.02.2022
(51) Int. Cl.: A61B 17/34, A61N 1/00, A61M 25/06, A61B 17/00

(54) **INTRODUCER TOOL**

(71) Applicant: BIOTRONIK SE & Co. KG, 12359 Berlin (DE)
(72) Inventor: Halank, Irina, 13189 Berlin (DE); Schippel, Mark, 15834 Rangsdorf (DE); Kolberg, Gernot, 12161 Berlin (DE)
(74) Representative: Biotronik Corporate Services SE

(57) **Abstract**

The invention relates to an introducer tool (1) for implanting an electrode into a human or animal body. The introducer tool (1) has a distal end and a proximal end and comprises: an elongated shaft (2) extending along a longitudinal shaft axis (L) between the proximal end and the distal end; a first handle (3) arranged at the proximal end and radially protruding from the elongated shaft (2) along a first handle axis (30), the first handle axis (30) extending from a first handle axis origin (31) lying at a contact surface between the first handle (3) and the elongated shaft (2) towards a first handle axis terminus (32) lying at a free end of the first handle (3); and a second handle (4) arranged at the proximal end and radially protruding from the elongated shaft (2) along a second handle axis (40), the second handle axis (40) extending from a second handle axis origin (41) lying at a contact surface between the second handle (4) and the elongated shaft (2) towards a second handle axis terminus (42) lying at a free end of the second handle (4). According to an aspect of the invention, the first handle axis (30) and the second handle axis (40) extend in a single radial plane with respect to the longitudinal shaft axis (L) and are at least at the first handle axis origin (31) and the second handle axis origin (41) angularly arranged to each other, wherein an angle (α, β, γ, δ, θ) between the first handle axis (30) at the first handle axis origin (31) and the second handle axis (40) at the second handle axis origin (41), measured along a circumference of the longitudinal shaft (2), lies in a range of from 10° to 170°.

## Description

The present invention relates to an introducer tool for implanting an electrode into a human or animal body according to the preamble of claim 1 and to an implantation assembly comprising such an introducer tool according to the preamble of claim 12.

Introducer tools comprising a peelable sheath, such as a peelable dilator sheath, typically comprise two or more handles to peel the sheath. However, the handle orientation is often chosen such that the handles interfere with the desired implantation path of an electrode to be implanted with the help of the peelable sheath. In many cases, one of the handles is removed from the peelable sheath by breaking it off to allow a better view onto the desired implantation path. However, this makes it more difficult to subsequently peel the sheath with the remaining handle.

Depending on the implantation conditions, the handles used for peeling a peelable sheath are not freely accessible. In particular if that side of the electrode to be implanted by an introducer tool that protrudes from the introducer tool (also referred to as introducer catheter) is no longer freely movable, but already implanted, tunneled or connected, it often runs directly over one of the peeling handles. As a consequence, this handle cannot be gripped any longer correctly since the electrode interferes with the handle.

Another problem are obese patients who require skin incisions that are often so deep that the peeling handles known from prior art and having a 180° orientation with respect to each other align themselves parallel to the patient surface. As a consequence, the handles disappear within the deep skin incision and are no longer visible for the operator. To avoid such undesired orientations of the introducing sheath, the operators often apply a different orientation of the sheath, which is, however, suboptimal with respect to an implantation of an electrode. In many instances, the skin incision also needs to be enlarged to allow a safe operation of the introducing sheath. This, however, hampers the healing process.

EP 3 042 686 A1 describes an introducer sheath comprising two handles which are oriented at an angle of 180° to each other. To allow an easy operation of these handles, they are additionally inclined towards a longitudinal axis of the introducer sheath. However, such an orientation of the handles makes it even more likely that the handles disappear within a skin incision and does not solve the above-mentioned problems. Other embodiments described in this European patent application make use of more than two handles in a butterfly-like arrangement. However, such an arrangement makes a visual control of the implantation site even more difficult and even more likely interferes with an electrode to be implanted by the introducer sheath.

It is an object of the present invention to provide an introducer sheath that comprises handles which are arranged in a more ergonomic way than according to prior art solutions, wherein the introducer sheath should allow an easier implantation of an electrode and a safe operation by an operator even in case of using the introducer sheath within a deep skin incision.

This object is achieved with an introducer tool for implanting an electrode into a human or animal body having the features of claim 1. Such an introducer tool has a distal end and a proximal end. It furthermore comprises an elongated shaft that extends along a longitudinal shaft axis between the proximal end and the distal end. The introducer tool further comprises a first handle and a second handle. Both the first handle and the second handle are arranged at the proximal end of the introducer tool. The first handle radially protrudes from the elongated shaft along a first handle axis. The first handle axis extends from a first handle axis origin, which is positioned at a contact surface between the first handle and the elongated shaft, towards a first handle axis terminus, which is positioned at a free end of the first handle. Likewise, the second handle radially protrudes from the elongated shaft along a second handle axis. The second handle axis extends from a second handle axis origin, which is positioned at a contact surface between the second handle and the elongated shaft, towards a second handle axis terminus, which is positioned at a free end of the second handle.

According to an aspect of the presently claimed invention, the first handle axis and the second handle axis extend in a single (and the same) radial plane with respect to the longitudinal shaft axis. Expressed in other words, neither the first handle nor the second handle are inclined towards the longitudinal shaft axis, but rather protrude from the longitudinal shaft at an angle of approximately 90° so that they only lie in a single radial plane. Different radial planes are arranged in parallel to each other along the longitudinal shaft axis. I.e., when virtually moving along the longitudinal shaft axis, one traverses a plurality of different radial planes.

The first handle axis and the second handle axis are at least at the first handle axis origin and the second handle axis origin angularly arranged to each other. In this respect, an angle between the first handle axis at the first handle axis origin and the second handle axis at the second handle axis origin lies in a range of from 10° to 170°, in particular of from 20° to 160°, in particular of from 30° to 150°, in particular of from 40° to 140°, in particular of from 50° to 130°, in particular of from 60° to 120°, in particular of from 70° to 110°, in particular of from 80° to 100°, in particular of from 90° to 95°. An angle between the first handle axis and the second handle axis lying in a range of from 90° to 120° is particularly appropriate. The angle is measured along the circumference of the elongated shaft. Thus, the angle does not indicate an inclination of the first handle and the second handle with respect to the longitudinal shaft axis, but rather a radial orientation of the first handle and the second handle at the elongated shaft. Expressed in other words, the angle is measured in a radial direction (about the longitudinal shaft axis), not in a direction traversing the longitudinal shaft axis.

Such an arrangement of the first handle and the second handle allows a particularly easy peeling of the elongated shaft once the device to be implanted (such as an electrode) has reached its intended site of implantation. Due to the angular handle arrangement deviating from 180° orientations known from prior art, the introducer sheath does not disappear within a skin incision, even if the skin incision needs to be deep due to obesity of the patient. Since the first handle and the second handle are already closer to each other than in case of prior art solutions, a bigger radial space is left free for guiding an electrode or another medical device to be implanted by the introducer sheath. This facilitates the whole implantation process since it is no longer necessary to break off one of the handles to obtain a good view onto the device to be implanted with the help of the introducer tool. During an implantation, the introducer tool can be rotated such that both handles are easily accessible. Even if an electrode to be implanted exits from the elongated shaft in a lateral way, the presently claimed orientation of the handles with respect to the elongated shaft allows an easy peeling of the shaft upon completion of the implantation process. In case of deep skin incisions in obese patients, the introducer tool can be rotated such that both the first handle and the second handle protrude from the performed skin incision and can thus be easily reached and operated.

In an embodiment, the first handle axis and/or the second handle axis has a non-straight course in the single radial plane. While a straight course of the first handle axis and/or the second handle axis (which is also claimed in an embodiment) allows a straight arrangement of the first handle and/or the second handle (e.g., in cuboid-like manner), a non-straight course of at least one of the handle axes also allows different shapes of the first handle and/or the second handle.

In an embodiment, the first handle axis and/or the second handle axis has a bent course in the single radial plane. Such a bent course allows a U-like shape of the first handle and/or the second handle. To determine an angle between the first handle axis and the second handle axis in case of such a bent course, a tangent can be laid alongside any point of the respective handle axis. An angle between the two tangents will then indicate the angle between the handle axes at the respective point.

In an embodiment, the first handle axis and/or the second handle axis has an angled course in the single radial plane. Such an angled course may be realized, e.g., by a course of a first straight axis section and a second straight axis section, wherein an angle is formed between the first straight axis section and the second straight axis section. By such an angled course of the first handle axis and/or the second handle axis, a V-like shape of the first handle and/or the second handle is made possible.

By shaping the first handle and/or the second handle in a U-like shape or a V-like shape, in particular ergonomic design of the first handle and/or the second handle is made possible that facilitates the operation of the introducer tool.

In an embodiment, the first handle has a first bulge at the free end of the first handle. Alternatively or additionally, the second handle has a second bulge at the free end of the second handle. The first bulge and/or the second bulge facilitate gripping of the handle and thus an operation of the introducer tool by an operator. The first bulge and/or the second bulge can be oriented to only one of the bottom or top side of the first handle or the second handle, respectively. Alternatively, the first bulge and/or the second bulge can also be oriented to both a top side and a bottom side of the first handle or the second handle, respectively. The thickness of the bulge can be adapted to the users' needs. From a constructional point of view, no specific restrictions apply to the size of the bulge.

In an embodiment, the first handle and the second handle are identically constructed. This allows a symmetric design of the introducer tool and a particularly intuitive operation of the introducer tool.

In an embodiment, the elongated shaft comprises a single predetermined breaking line that extends along the longitudinal shaft axis. This predetermined breaking line serves for an easy peeling of the elongated shaft after completion of the implantation process. A single predetermined breaking line is particularly helpful for such a peeling process if the first handle and the second handle are arranged quite closely to each other (e.g., in a radial angle of 10° to 90°, in particular of 20° to 80°, in particular of 30° to 70°, in particular of 40° to 60°). Then, both the first handle and the second handle can be gripped with a single hand of the operator and radially pressed against each other, leading in a rupturing of the predetermined breaking line and a peeling of the elongated shaft.

In an embodiment, the single predetermined breaking line is arranged in a region of the circumference of the longitudinal shaft that is most distant to both the first handle and the second handle. By such an arrangement, the distance between the single predetermined breaking line and the first handle on the one side as well as the distance between the predetermined breaking line and the second handle on the other side is equally big. Then, forces applied to the first handle and the second handle will be equally transferred to the predetermined breaking line, leading in an easy and reliable rupture of the predetermined breaking line.

In an embodiment, the elongated shaft comprises two predetermined breaking lines extending along the longitudinal shaft axis. If two of these predetermined breaking lines are provided, the elongated shaft can be divided into two separate parts. This may facilitate the removal of the elongated shaft from an implantation site under certain circumstances. In addition, the forces necessary to achieve a rupturing of the individual predetermined breaking lines may be smaller than in case of providing only a single predetermined breaking line.

In an embodiment, each of the two predetermined breaking lines is arranged in a separate region of the circumference of the longitudinal shaft, wherein the regions lie opposite (i.e., at an angle of approximately 180°) to each other. By such an arrangement, a particularly easy rupturing of the predetermined breaking lines can be achieved. Furthermore, the elongated shaft will then be divided into two identical parts upon completion of the peeling process.

In an embodiment, the introducer tool comprises exactly two handles, i.e., no other handle than the first handle and the second handle. These two handles are fully sufficient for a safe operation of the introducer tool. A third handle would rather impede safe operation of the introducer tool and would hinder a free view onto the operation space.

In an aspect, the present invention relates to an implantation assembly comprising an introducer tool according to the preceding explanations as well as an electrode of an implantable medical device. This electrode is to be implanted with the help of the introducer tool. Upon implantation of the electrode to its intended site of implantation, the elongated shaft of the introducer tool will be typically peeled by pulling the first handle and the second handle towards the distal end of the introducer tool. The elongated shaft can then be removed either as a single part or as more than one part (e.g., two parts), wherein the electrode of the implantable medical device remains at the intended site of implantation.

In an embodiment, the electrode is an electrode of an implantable pulse generator (IPG), an electrode of an implantable cardioverter-defibrillator (ICD), an electrode of a device for cardiac resynchronization therapy (CRT), or an electrode of an implantable cardiac monitor.

All embodiments of the introducer tool can be combined in any desired manner and can be transferred either individually or in any arbitrary combination to the implantation assembly. Likewise, all embodiments of the implantation assembly can be combined in any desired manner and can be transferred either individually or in any arbitrary combination to the introducer tool.

Further details of aspects of the present invention will be explained in the following making reference to exemplary embodiments and accompanying Figures. In the Figures:
- Fig. 1A: shows a top view onto a first embodiment of an introducer tool;
- Fig. 1B: shows a side view onto the proximal end of the introducer tool of Fig. 1A;
- Fig. 2A: shows a top view onto a second embodiment of an introducer tool;
- Fig. 2B: shows a side view onto the proximal end of the introducer tool of Fig. 2A;
- Fig. 3A: shows a top view onto a third embodiment of an introducer tool;
- Fig. 3B: shows a side view onto the proximal end of the introducer tool of Fig. 3A;
- Fig. 4A: shows a top view onto a fourth embodiment of an introducer tool;
- Fig. 4B: shows a top view onto a fifth embodiment of an introducer tool;
- Fig. 5A: shows a top view onto a sixth embodiment of an introducer tool in a first operational state; and
- Fig. 5B: shows a top view onto the introducer tool of Fig. 5A in a second operational state.

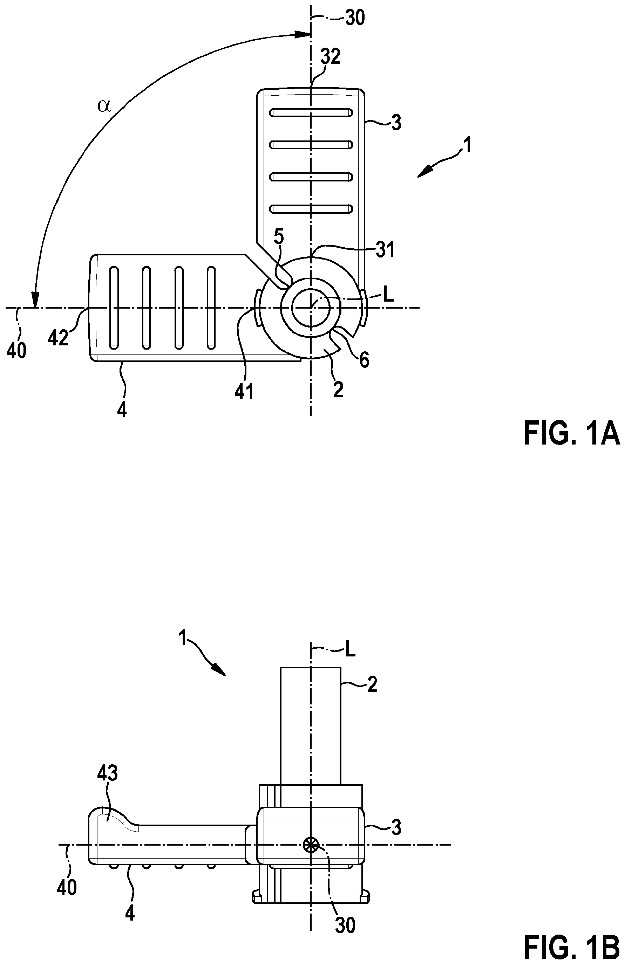

Fig. 1A shows a top view onto the proximal end of an introducer sheath 1 serving as introducer tool. The introducer sheath 1 comprises an elongated shaft 2 that extends along a longitudinal shaft axis L. The introducer sheath 2 further comprises a first handle 3 and a second handle 4.

The first handle 3 radially protrudes from the elongated shaft 2 along a first handle axis 30. The first handle axis 30 extends between the first handle axis origin 31 and a first handle axis terminus 32. The first handle axis origin 31 is located at a contact surface between the first handle 3 and the elongated shaft 2. The first handle axis terminus 32 is located at a free end of the first handle 3.

Likewise, the second handle 4 protrudes from the elongated shaft 2 in a radial way along a second handle axis 40. The second handle axis 40 extends from a second handle axis origin 41 to a second handle axis terminus 42. The second handle axis origin 41 is located at a contact surface between the second handle 4 and the elongated shaft 2. The second handle axis terminus 42 is located at a free end of the second handle 4.

In the embodiment shown in Fig. 1A, an angle α between the first handle axis 30 and the second handle axis 40 amounts to 90°. Due to this angular arrangement between the first handle 3 and the second handle 4, a big area of the elongated shaft 2 remains unaffected from the first handle 3 and the second handle 4 so that an electrode which is guided within the elongated shaft 2 along the longitudinal axis L can be guided in this area and does not interfere with the first handle 3 nor the second handle 4.

Once the electrode (or the other medical device that is to be introduced into a human or animal body with the help of the introducer sheath 1) is implanted at its intended site of implantation, the first handle 3 and the second handle 4 can be used to break off a first predetermined breaking line 5 and a second predetermined breaking line 6 so as to peel the elongated shaft 2 around the implanted electrode or other medical device. As a result, the introducer sheath 1 can be removed from the implanted electrode or other medical device that remains at its intended site of implantation.

Fig. 1B shows a lateral view onto the introducer sheath 1 of Fig. 1A. In this depiction, the proximal end of the introducer sheath 1 is facing downwards, wherein the elongated shaft 2 that extends to the distal end of the introducer sheath 1 (which distal and is not shown in Fig. 1B) is facing upwards. In this and in all following Figures, similar elements will be denoted with the same numeral reference.

It is apparent from the depiction of Fig. 1B that the first handle 3 and the second handle 4 lie in the same radial plane.

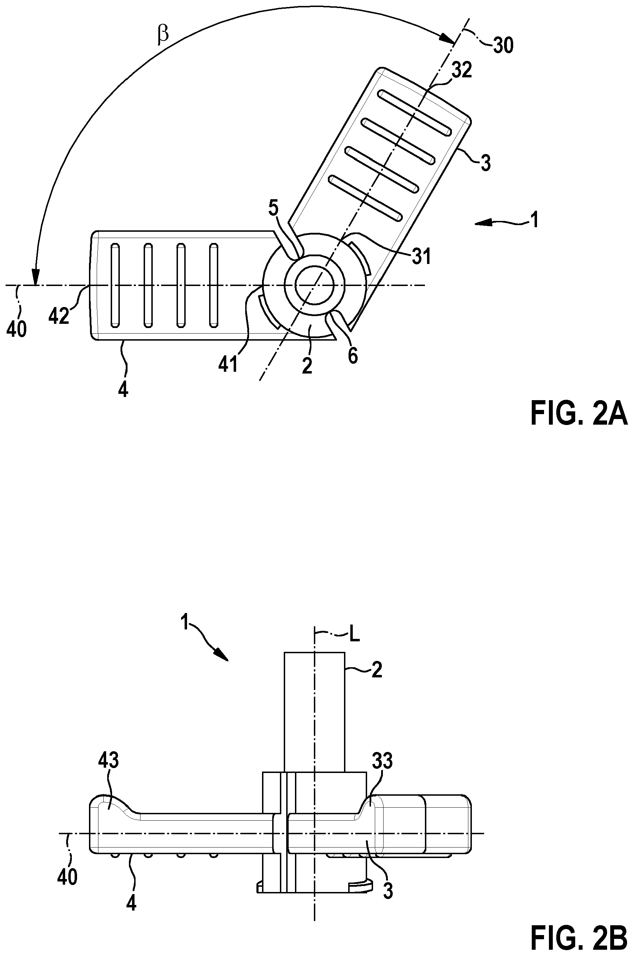

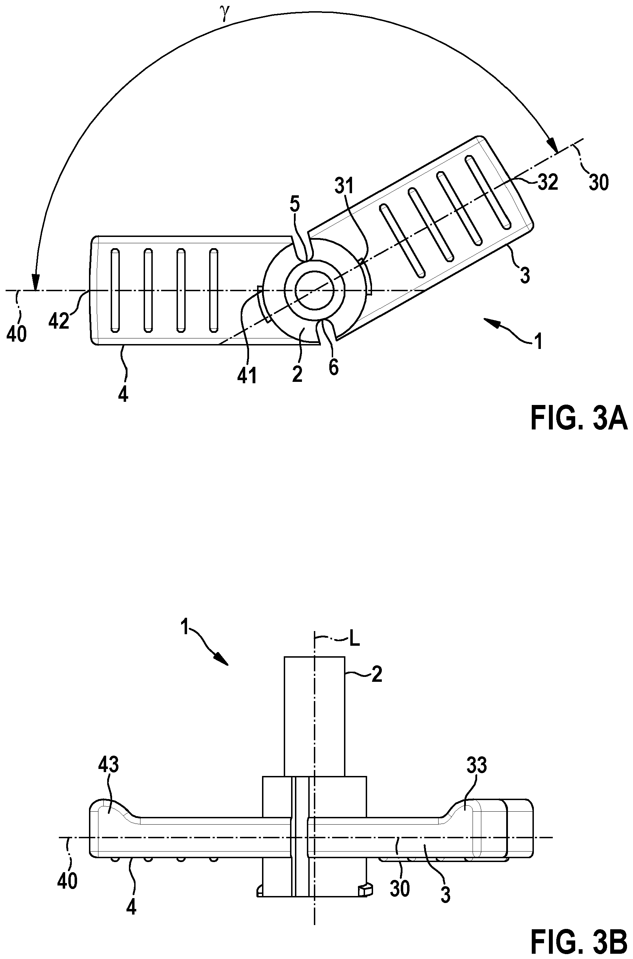

The second handle 4 comprises a bulge 43 at its bottom site (facing upwards in the depiction of Fig. 1B). This bulge 43 facilitates gripping the handle 4 by a human operator. Likewise, the first handle 3 comprises such a bulge at its bottom side. Due to the perspective of depiction, this bulge of the first handle 3 can hardly be seen in Fig. 1B. However, reference is made in this respect to the explanations given with respect Figures 2B and 3B below.

Fig. 2A shows another embodiment of an introducer sheath 1 in a top view onto the proximal end of the introducer sheath 1. This embodiment is very similar to the embodiment shown in Fig. 1A so that reference is made to the explanations given above with respect to Fig. 1A. The only difference is that the first handle axis 30 and the second handle axis 40 are arranged to each other at an angle β amounting to 120°. Since this angle β is somewhat bigger than the angle α of the embodiment shown in Fig. 1A, the region around the second predetermined breaking line 6 which remains un-influenced by the first handle 3 and the second handle 4 is slightly smaller than in case of the embodiment of Fig. 1A. As a consequence, the space remaining for an electrode or another medical device to be implanted by the introducer sheath 1 is somewhat smaller. Nonetheless, an electrode or other medical device to be implanted by the introducer sheath 1 can still be safely guided in this region without interfering with the first handle 3 or the second handle 4.

Fig. 2B shows a lateral view onto the embodiment of Fig. 2A, wherein the perspective is the same as in case of Fig. 1B. In the depiction of Fig. 2B, the bulge 33 on the bottom site (facing upwards in the depiction of Fig. 2B) of the first handle 3 can be well seen. It is constructed identically to the bulge 43 of the second handle 4.

Fig. 3A shows another embodiment of an introducer sheath 1 that is very similar to the embodiments of Fig. 1A and Fig. 2A. Therefore, reference is made to the explanations given above. Once again, Fig. 3A is a top view onto this embodiment of the introducer sheath 1. The only difference to the embodiments shown in Figures 1A and 2A is that the first handle 3 and the second handle 4 are arranged to each other at an angle γ amounting to 150°. Even this big angle of 150° allows a better view onto the region around the second predetermined breaking line 6 through which an electrode or another medical device to be implanted by the introducer sheath 1 can be guided. Therefore, also this embodiment provides an advantage with respect to prior art introducer sheaths employing a 180° arrangement of two handles.

Fig. 3B shows a lateral view onto the embodiment of the introducer sheath 1 of Fig. 3A. The same perspective depiction as in Figures 1B and 2B is chosen so that the proximal end of the introducer sheath 1 is facing downwards.

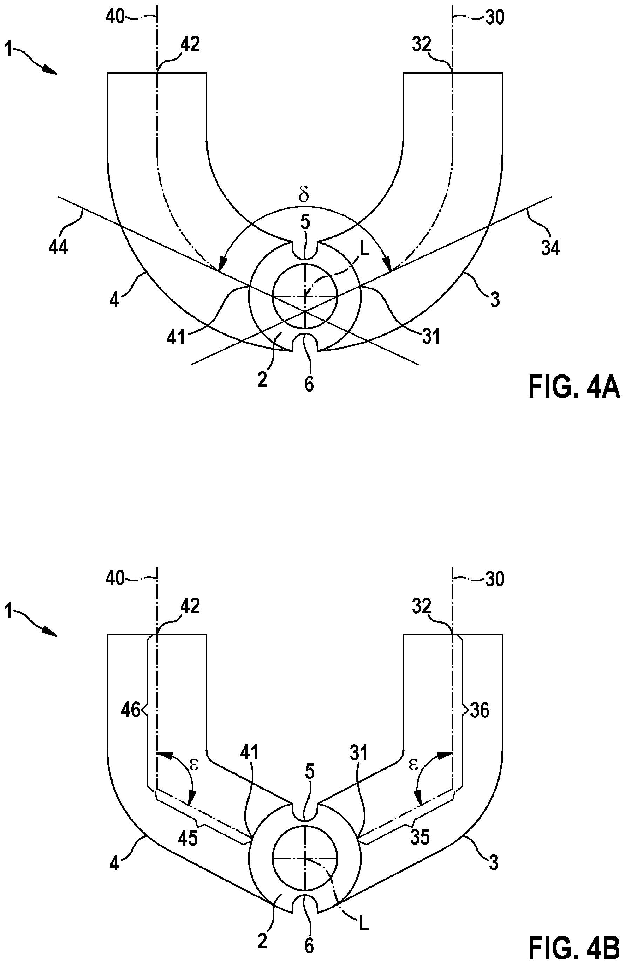

Fig. 4A shows another embodiment of an introducer sheath 1, wherein the first handle 3 and the second handle 4 together have a U-like shape. The first handle axis 30 and the second handle axis 40 have both a bent course. By laying a first tangent 34 alongside the first handle axis 30 at the first handle axis origin 31 and a second tangent 44 alongsinde the second handle axis 40 at the second handle axis origin 41, an angle δ between the first tangent 34 and the second tangent 44 can be measured. It reflects the radial angle between the first handle axis 30 and the second handle axis 40 at the first handle axis origin 31 and the second handle axis origin 41. The angle δ amounts to approximately 120° in the embodiment shown in Fig. 4A.

The other details of the introducer sheath 1 depicted in Fig. 4A are similar or even identical to the embodiments shown in Figures 1A to 3B so that reference is made to the explanations given above.

Fig. 4B shows another embodiment of an introducer sheath 1 that is similar to the embodiment shown in Fig. 4A. The only difference between the embodiments shown in Figures 4A and 4B is the course of the first handle axis 30 and the second handle axis 40. In the embodiment shown in Fig. 4B, the first handle axis 30 and the second handle axis 40 have an angled course. Consequently, also the first handle 3 and the second handle 4 have an angled shape, resulting in a V-like shape when taken the first handle 3 and the second handle 4 together. The angled course of the first handle axis 30 is achieved by a straight first handle axis section 35 and a straight second handle axis 36 that are angularly arranged to each other at an angle ε amounting to approximately 125°. Likewise, the second handle axis 40 comprises a straight third handle axis section 45 and a straight fourth handle axis section 46 that are also angularly arranged to each other at the same angle ε.

Regarding all other elements of the introducer sheath 1, reference is made to the explanations given above with respect to Figures 1A to 4A.

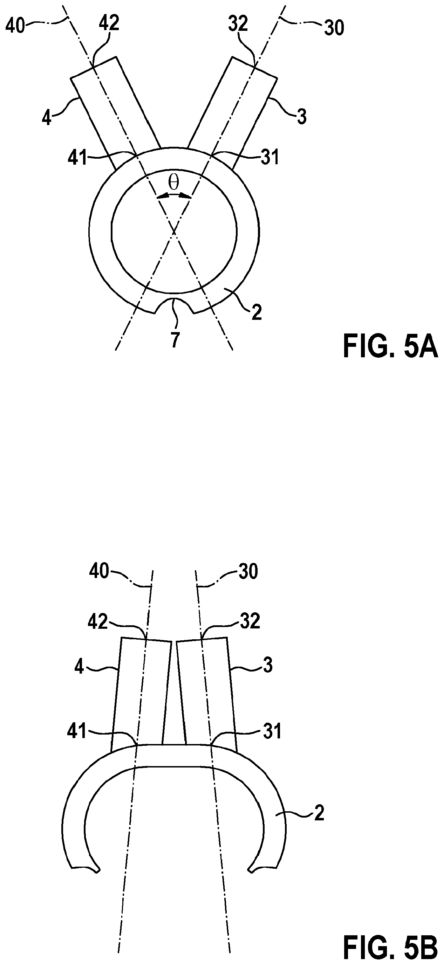

Fig. 5A shows a top view onto another embodiment of an introducer sheath 1 that is similar to the previously explained embodiments. In contrast to the previously explained embodiments, the embodiment of Fig. 5A has only a single predetermined breaking line 7 instead of the first predetermined breaking line 5 and the second predetermined breaking line 6. As a consequence, the elongated shaft 2 cannot be fully broken into two separate parts. Rather, it can only be opened by rupturing the single predetermined breaking line 7. To achieve such a rupturing, the first handle 3 and the second handle 4 are pressed towards each other along the circumference of the elongated shaft 2. To allow an easy relative movement of the first handle 3 and the second handle 4 with respect to each other, the first handle axis 30 and the second handle axis 40 include an angle θ amounting to approximately 50°.

Fig. 5A shows the introducer sheath 1 in an operational state in which the single predetermined breaking line 7 is still intact. In Fig. 5B, a top view on the same introducer sheath 1 as in Fig. 5A is shown. In this depiction, however, the single predetermined breaking line 7 has already been ruptured by moving the first handle 3 and the second handle 4 towards each other. In this operational state, the introducer sheath 1 can be removed from an electrode or other medical device that has been implanted with the help of the introducer sheath into a human or animal body.

Due to the small radial angle between the first handle 3 and the second handle 4 of the embodiment shown in Figures 5A and 5B, it is possible to operate this introducer sheath 1 with a single hand. This facilitates an implantation procedure making use of the introducer sheath 1.

## Claims

1. Introducer tool (1) for implanting an electrode into a human or animal body, the introducer tool (1) having a distal end and a proximal end and comprising:
an elongated shaft (2) extending along a longitudinal shaft axis (L) between the proximal end and the distal end;
a first handle (3) arranged at the proximal end and radially protruding from the elongated shaft (2) along a first handle axis (30), the first handle axis (30) extending from a first handle axis origin (31) lying at a contact surface between the first handle (3) and the elongated shaft (2) towards a first handle axis terminus (32) lying at a free end of the first handle (3);
a second handle (4) arranged at the proximal end and radially protruding from the elongated shaft (2) along a second handle axis (40), the second handle axis (40) extending from a second handle axis origin (41) lying at a contact surface between the second handle (4) and the elongated shaft (2) towards a second handle axis terminus (42) lying at a free end of the second handle (4);
**characterized**
**in that** the first handle axis (30) and the second handle axis (40) extend in a single radial plane with respect to the longitudinal shaft axis (L) and are at least at the first handle axis origin (31) and the second handle axis origin (41) angularly arranged to each other, wherein an angle (α, β, γ, δ, θ) between the first handle axis (30) at the first handle axis origin (31) and the second handle axis (40) at the second handle axis origin (41), measured along a circumference of the longitudinal shaft (2), lies in a range of from 10° to 170°.

2. Introducer tool according to claim 1, **characterized in that** at least one of the first handle axis (30) and the second handle axis (40) has a non-straight course in the single radial plane.

3. Introducer tool according to claim 1 or 2, **characterized in that** at least one of the first handle axis (30) and the second handle axis (40) has a bent course in the single radial plane.

4. Introducer tool according to claim 1 or 2, **characterized in that** at least one of the first handle axis (30) and the second handle axis (40) has an angled course in the single radial plane.

5. Introducer tool according to any of the preceding claims, **characterized in that** the first handle (3) has a first bulge (33) at the free end of the first handle (3) and/or **in that** the second handle (4) has a second bulge (43) at the free end of the second handle (4).

6. Introducer tool according to any of the preceding claims, **characterized in that** the first handle (3) and the second handle (4) are identically constructed.

7. Introducer tool according to any of the preceding claims, **characterized in that** the elongated shaft (2) comprises a single predetermined breaking line (7) extending along the longitudinal shaft axis (L).

8. Introducer tool according to claim 7, **characterized in that** the single predetermined breaking line (7) is arranged in a region of the circumference of the longitudinal shaft (2) that is most distant to both the first handle (3) and the second handle (4).

9. Introducer tool according to any of claims 1 to 6, **characterized in that** the elongated shaft (2) comprises two predetermined breaking lines (5, 6) extending along the longitudinal shaft axis (L).

10. Introducer tool according to claim 9, **characterized in that** each of the two predetermined breaking lines (5, 6) is arranged in a separate region of the circumference of the longitudinal shaft (2), wherein the regions lie opposite to each other.

11. Introducer tool according to any of the preceding claims, **characterized in that** the introducer tool (1) comprises no other handle than the first handle (3) and the second handle (4).

12. Implantation assembly comprising an introducer tool (1) according to any of the preceding claims and an electrode of an implantable medical device.

13. Implantation assembly according to claim 12, **characterized in that** the electrode is an electrode of an implantable pulse generator, of an implantable cardioverter-defibrillator, of a device for cardiac resynchronization therapy, or of an implantable cardiac monitor.
